(19) **Europäisches Patentamt European Patent Office Office européen des brevets**

(11) **EP 1 872 811 B1**

(12) **EUROPEAN PATENT SPECIFICATION**

(45) Date of publication and mention
of the grant of the patent:
**06.04.2011 Bulletin 2011/14**

(21) Application number: **06714866.8**

(22) Date of filing: **28.02.2006**

(51) Int Cl.:
***A61M 1/14*** (2006.01)

(86) International application number:
**PCT/JP2006/303729**

(87) International publication number:
**WO 2006/112154 (26.10.2006 Gazette 2006/43)**

(54) **Hemodialyzer**

Hämodialysator

Appareil d'hémodialyse

(84) Designated Contracting States:
**DE FR GB IT SE**

(30) Priority: **08.04.2005 JP 2005112552**

(43) Date of publication of application:
**02.01.2008 Bulletin 2008/01**

(73) Proprietor: **NIKKISO COMPANY, LTD.
Shibuya-ku,
Tokyo 150-8677 (JP)**

(72) Inventors:
• **MORI, Yoshihiro,
c/o Nikkiso Company Limited
Makinohara-shi,
Shizuoka 421-0496 (JP)**

• **TOYODA, Masahiro,
c/o Nikkiso Company Limited
Makinohara-shi,
Shizuoka 421-0496 (JP)**

(74) Representative: **GROSSE SCHUMACHER
KNAUER VON HIRSCHHAUSEN
Patent- und Rechtsanwälte
Nymphenburger Strasse 14
80335 München (DE)**

(56) References cited:
**EP-A1- 1 205 144      EP-A1- 1 364 666
WO-A1-02/13691      JP-A- 2004 049 492
JP-A- 2004 505 708      US-A- 5 938 938**

• **TABEI K. ET AL.: 'Josui ni yoru Tanpaku Noshukudo no Igi no kento' JOURNAL OF JAPANESE SOCIETY FOR DIALYSIS THERAPY vol. 32, no. 7, 1999, pages 1071 - 1077**

**Description**

TECHNICAL FIELD

The present invention relates to a hemodialysis apparatus which performs hemodialysis and ultrafiltration by extracorporeally circulating blood of a patient.

BACKGROUND ART

Generally, a hemodialysis treatment uses a hemodialysis apparatus that includes a blood circuit for extracorporeally circulating blood of a patient, a dialyzer provided at an intermediate portion of the blood circuit, a peristaltic type blood pump, and a dialysis device body for performing hemodialysis treatment by inletting and outletting dialysate from and to the dialyzer while ultrafiltrating. Note that; the blood circuit primarily includes an arterial blood circuit having an arterial needle at end thereof, and a venous blood circuit having a venous needle at an end thereof.

When the arterial needle and the venous needle are inserted into a patient and the blood pump is driven, blood of the patient is introduced into the blood circuit through the arterial needle, and then returns into the body of the patient through the arterial blood circuit, the dialyzer and the venous blood circuit. The dialyzer includes a plurality of hollow fibers forming hemodialysis membranes. The blood flows inside of the hollow fibers, and the dialysate with a predetermined concentration supplied from the dialysis device body flows outside (i.e., between the outer surfaces of the hollow fibers and the inner surface of the body of the dialyzer) of the hollow fibers so that waste products within the blood flowing through the inside of the hollow fibers permeate into the dialysate through the hemodialysis membrane.

Accordingly, the blood from which waste products are removed returns into the body of the patient through the venous blood circuit. On the other hand, the dialysis device body is provided with an ultrafiltration pump for removing water from blood of a patient so that the blood is ultrafiltrated through the hemodialysis membranes during the hemodialysis treatment. A volume of water (ultrafiltration rate) to be ultrafiltrated by the ultrafiltration pump is adjusted by controlling driving of the ultrafiltration pump.

An ultrafiltration volume controlled by the above-mentioned ultrafiltration pump is to be set so that the body weight of the patient becomes close to a dry-weight (a body weight of a patient when a volume of an interstitial fluid outside of cells is properly adjusted by the hemodialysis treatment) of the patient. In this regard, the dry-weight is generally calculated based on experiences of a medical staff such as a medical doctor, who relates the dry-weight to parameters such as a cardiothoracic index, changes in blood pressures during the hemodialysis treatment, variation in a blood benchmark (a variation rate of a circulating blood volume ΔBV, for example), the body weight of the patient before the hemodialysis treatment, and a decrease of the body weight during the hemodialysis treatment. Note that, such a prior art does not accord to inventions opened to the public, so that there is no information of prior art documents.

An example of prior art devices is given in US-5938938.

SUMMARY OF THE INVENTION

PROBLEMS TO BE SOLVED BY THE INVENTION

In the above-described hemodialysis apparatus, since the ultrafiltration volume is determined based on the dry-weight calculated based on an experiential rating by a medical staff, there is a problem in that the ultrafiltration volume for each patient is not necessarily accurate due to differences in physiques and in a blood benchmark. That is, it is ideal to perform the ultrafiltration until the body weight of the patient reaches an accurate dry-weight of the patient, however the calculation of the dry-weight is much depended on knowledge and experiences of a medical staff and, as occasion demands, there is a fear that an inappropriate dry-weight will be determined.

In view of the foregoing circumstances, the present invention is intended to provide a hemodialysis apparatus for hemodialysis which can perform an appropriate ultrafiltration by relating a predetermined blood-benchmark to the body weight of the patient under the hemodialysis treatment and calculating standardized parameters applicable to a large number of patients in conjunction with the dry-weight of the patient.

MEANS FOR SOLVING THE PROBLEM

The invention is described in claim 1.

Dependent claims 2 to 8 refer to further embodiments of the apparatus described in claim 1.

EFFECT OF THE INVENTION

According to the invention, since a standardized parameter applicable to a large number of patients relating a predetermined blood benchmark to a body weight of a patient during a hemodialysis treatment in correlation with a dry-weight of the patient is calculated during the hemodialysis treatment, the parameter can be treated as a benchmark to perform in real-time an optimal ultrafiltration.

According to the embodiment of claim 2, since the parameter is calculated by using a variation rate of the body weight obtained based on the body weight of the patient before the hemodialysis treatment and a volume of the ultra-filtration, and a blood benchmark (a variation rate of a volume of circulating blood plasma) based on a concentration of extracorporeally circulating blood, the parameter as a benchmark during the hemodialysis treatment can be smoothly obtained in real-time utilizing the existing hemodialysis apparatus without any modification.

According to the embodiment of claim 3, since the calculated parameter is displayed on a display means and the like, it is possible for a medical staff such as a doctor to recognize in real-time whether or not an optimal ultrafiltration is performed, thus favorably performing an optimal during the hemodialysis treatment.

According to the embodiment of claim 4, since a change of the calculated parameter with respect to time is graphically displayed, a medical staff can perceive a transition and trend of the parameter, thus further performing an optimal ultrafiltration during the hemodialysis treatment

According to the embodiment of claim 5, since an optimal range of the parameter is set and a state of the parameter is informed when the calculated parameter has deviated from the optimal range, there is no need to constantly monitor the parameter for medical staffs, thus improving working properties during the hemodialysis treatment.

According to the embodiment of claim 6, since an optimal range of the parameter is set and the dialysis treatment means and the like are controlled so that a value of the parameter becomes a value within the optimal range when a value of the calculated parameter deviates from the optimal range of the parameter, thus automatically performing an optimal ultrafiltration during the hemodialysis treatment.

According to the embodiment of claim 7, since the calculated parameter is stored and the stored parameter or a change of the stored parameter with respect to time can be displayed during other hemodialysis treatment or before or after the hemodialysis treatment, displaying of the parameter and its change with time of the identical patient during a hemodialysis treatment following the prior treatment allows medical staffs to study a trend of a mid-term or long term treatment and current conditions of the treatment. Also, displaying of the parameter and its change with time during a hemodialysis treatment for another patient allows medical staffs to study a comparison between patients. Further, displaying of the parameter and its changes with time of the parameter before or after a hemodialysis treatment assists medical staffs to explain to the patient the result of treatments with a comparison between current indications of the parameter and its changes with time and prior indications of them, or to explain a treatment policy in the future.

According to the embodiment of claim 8, since the stored parameter can be transmitted to an external terminal, the data can be stored, displayed and compared with related data in the external terminal. In this case, data of patients can be easily shared, and the shared data of patients can be arranged in a data base, which allows a collective management.

BEST MODE OF CARRYING OUT THE INVENTION

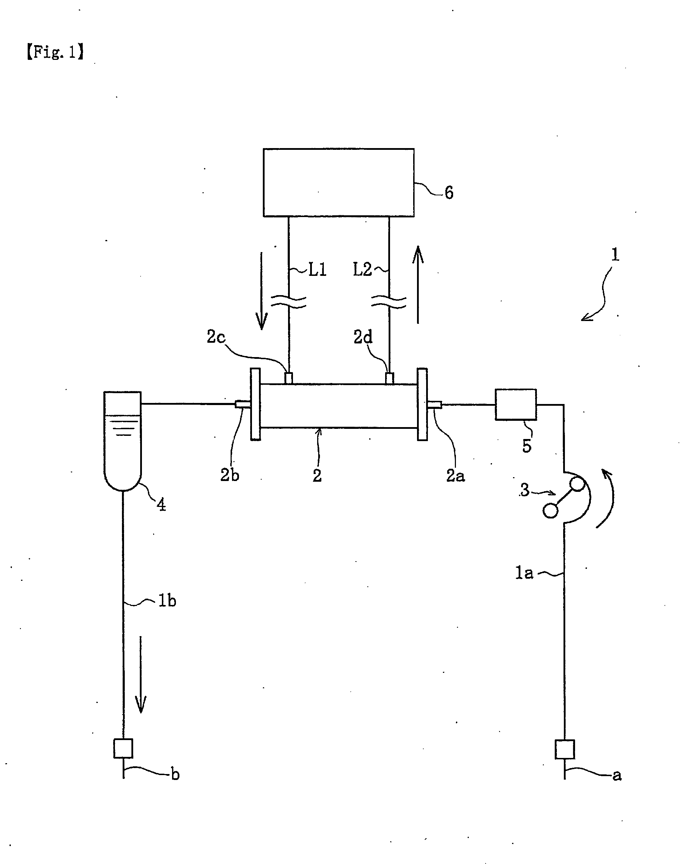

Hereinafter, a mode of carrying out the present invention will be explained in detail with reference to the accompanying drawings.
As shown in Fig. 1, a hemodialysis apparatus according to the present invention is used to perform hemodialysis and ultrafiltration by extracorporeally circulating blood of a patient and is mainly constructed by a blood circuit 1 for extracorporeally circulating the blood of the patient, a dialyzer 2 connected to the blood circuit 1 for a hemodialysis treatment, and a dialysis device body 6 connected to the dialyzer 2 for ultrafiltration of the blood while supplying dialysate. As shown in Fig. 1, the blood circuit 1 is mainly constructed by an arterial blood circuit 1a and a venous blood circuit 1b each formed by flexible tube. The dialyzer 2 is connected between the arterial blood circuit 1a and the venous blood circuit 1b.

The arterial blood circuit 1 a is provided with an arterial needle "a" at an end portion of the arterial blood circuit, and also provided with a roller-type blood pump 3 and a hematocrit sensor 5 in the middle of the arterial blood circuit. The venous blood circuit 1b is provided with a venous needle "b" at an end portion thereof, and a drip chamber 4 for removing bubbles in the middle thereof

The hematocrit sensor 5 is provided with a light emitting element such as an LED and a light receiving element such as a photodiode. The hematocrit sensor 5 irradiates the blood with a light of a predetermined wavelength from the light emitting element, and receives a transmitted or reflected light by the light receiving element to measure a hematocrit value indicating a concentration of the blood flowing through the blood circuit 1. That is, the hematocrit value is an index indicating a concentration of blood, and is expressed by a ratio of a volume of red blood cells to a volume of whole blood.

When the blood pump 3 is driven while the arterial needle "a" and the venous needle "b" are inserted into the patient, the blood of the patient flows through the arterial blood circuit 1a into the dialyzer 2 where the blood is purified, and flows into the drip chamber 4 where bubbles within the blood are removed, and then returns to the body of the patient through the venous blood circuit 1b. Thus, the blood of the patient is dialyzed by the dialyzer 2 during extracorporeal circulation through the blood circuit 1.

The dialyzer 2 is provided with a blood inlet port 2a, a blood outlet port 2b, a dialysate inlet port 2c and a dialysate outlet port 2d at a body thereof. The blood inlet port 2a and the blood outlet port 2b are each connected to ends of the arterial blood circuit 1a and the venous blood circuit 1b, respectively. Additionally, the dialysate inlet port 2c and the dialysate outlet port 2d are each connected to a dialysate inlet line L1 and a dialysate outlet line L2, respectively, both extending from the dialysis device body 6.

The dialyzer 2 includes a plurality of hollow fibers. The blood flows through the inside of the hollow fibers, and the dialysate flows through a portion between the outer surfaces of the hollow fibers and the inner surface of the body member of the dialyzer 2. In the hollow fibers, a large number of micro holes (pores) penetrating between the inner and outer surfaces of the hollow fibers are formed to form permeable membranes where impurities and the like in the blood are permeated into the dialysate.

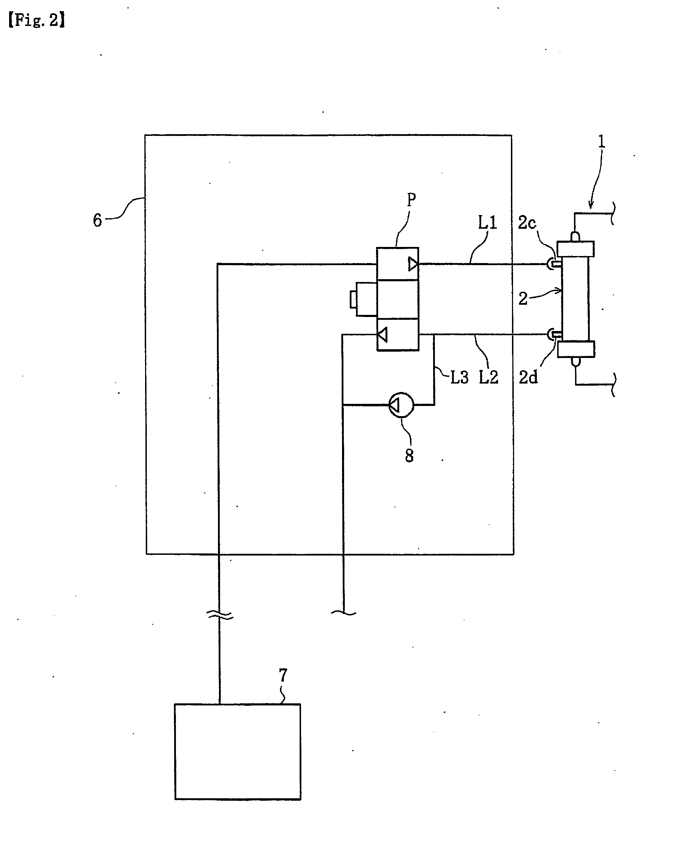

As shown in Fig. 2, the dialysis device body 6 is mainly constructed by a duplex pump P formed bridging the dialysate inlet line L1 and the dialysate outlet line L2, a bypass line L3 bypassing the duplex pump P in the dialysate outlet line L2, and an ultrafiltration pump 8 connected to the bypass line L3. One end of the dialysate inlet line L1 is connected to the dialyzer 2 (the dialysate inlet port 2c), and another end is connected to a dialysate supplying device 7 for producing a predetermined concentration of dialysate.

One end of the dialysate outlet line L2 is connected to the dialyzer 2 (the dialysate outlet port 2d), and another end is connected to a fluid disposal device (not shown) so that the dialysate, supplied from the dialysate supplying device 7 flows through the dialysate inlet line L1 into the dialyzer 2 and then flows through the dialysate outlet line L2 and the bypass line L3 into the fluid disposal device.

The ultrafiltration pump 8 is used for removing water from the blood of a patient flowing through the dialyzer 2. When the ultrafiltration pump 8 is driven, the duplex pump P is of a quantitative type, so that a volume of the dialysate flowing through the dialysate outlet line L2 from the dialyzer becomes greater than that of the dialysate flowing through the dialysate inlet line L1 into the dialyzer. Accordingly, the amount of quantity of water corresponding to the difference between the former volume of the dialysate and the latter volume of the dialysate is removed from the blood. Note that, devices other than the ultrafiltration pump 8 (a so-called balancing chamber, for example) may be used for removing water from the blood of a patient. Note that, the duplex pump P and the ultrafiltration pump 8 form a dialysis treatment means for performing hemodialysis treatment and ultrafiltration for an extracorporeally-circulated blood of a patient.

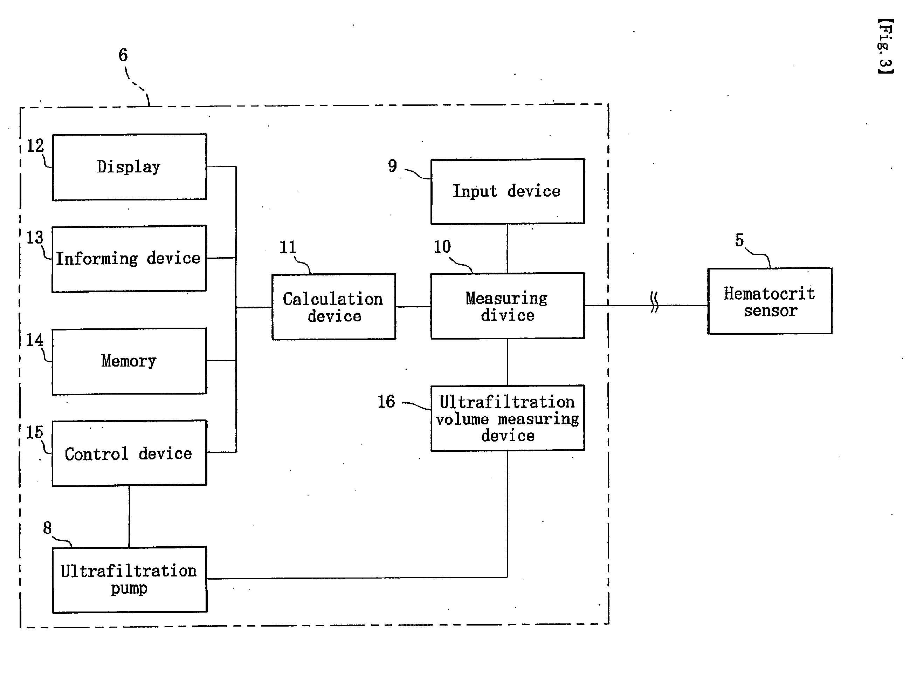

As shown in Fig. 3, the dialysis device body 6 is provided with an input means 9 for inputting a body weight of a patient measured before performing the hemodialysis treatment, a measuring means 10, a calculation means 11, a display means 12 formed by a display unit provided in the dialysis device body 6, an informing means 13 formed by such as a speaker capable of outputting audio signals, a storage means 14, a control means 15, and an ultrafiltration volume calculation means 16 for calculating the amount of removed-water based on a driving amount of the ultrafiltration pump 8.

The measuring means 10 is used for measuring a variation rate of the body weight of a patient under the hemodialysis treatment, and a variation rate of a volume of a circulating blood plasma as a predetermined blood benchmark, and is electrically connected to the input means 9, the control means 15 and the hematocrit sensor 5. Specifically, a variation rate of a body weight $\Delta BW\%$ is obtained by the following formula 1 by using a body weight of a patient (BW1) measured before the hemodialysis treatment, which is inputted by using the input means 9, and an ultrafiltration volume (an accumulated ultrafiltration volume UFV, at the time of the measurement) obtained by using the ultrafiltration volume calculation means 16 based on the driving rate of the ultrafiltration pump 8.

$$\Delta BW\% = (BW2 - BW1) / BW1 \times 100(\%)$$
$$= (-UFV) / BW1 \times 100(\%) \quad \text{--- (formula 1)}$$

It is noted that BW2 represents a body weight of a patient at the time of measurement by the measuring means 10, and increase and decrease of the body weight of a patient due to such as intake of food and excretion are disregarded.

The variation rate of a volume of the circulating blood plasma ($\Delta CPV\%$) (a variation rate of a blood benchmark) can be obtained by using a hematocrit value (Ht1%) measured at the time the ultrafiltration is started and a hematocrit value (Ht2%) measured at the time of measurement by the measuring means 10, into formula 2 which will be stated later. When CPV1 and BV1 are set to be a volume of circulating blood plasma and a volume of circulating blood at the time the ultrafiltration is started, respectively, CPV2 and BV2 are set to be a volume of circulating blood plasma and a volume of circulating blood at the time of measurement by the measuring means 10, respectively, and 1 (L) of blood = 1 (kg), since a volume of blood plasma = (a volume of blood) x ((1 - (ratio of constituent parts of blood)), CPV1 = BV1 x (1-Ht1 /100) and CPV2 = BV2 x (1 - Ht2 /100).

$$\Delta CPV\% = (CPV2 - CPV1) / CPV1 \text{ x } 100$$
$$= \{BV2\,(1 - Ht2 / 100) - BV1\,(1 - Ht1 / 100)\} /$$
$$\{BV1\,(1 - Ht1 / 100)\} \text{ x } 100$$
$$= (BV2 - BV1 - BV2 \text{ x } Ht2 / 100 + BV1 \text{ x } Ht1 / 100) / \{BV1\,(1 - Ht1 / 100)\} \text{ x } 100$$

In this regard, since red blood cells in the circulating blood are not reduced and the volume is consistent in the hemodialysis treatment, BV1 x Ht1 = BV2 x Ht2 (both sides indicate the volume of red cells in the volume of the circulating blood). Accordingly,

$$\Delta CPV\% = (BV2 - BV1) / \{BV1\,(1 - Ht1 / 100)\} \text{ x } 100 = (BV2 / BV1 - 1) /$$
$$(1 - Ht1 / 100) \text{ x } 100$$
$$= (Ht1 / Ht2 - 1) / (1 - Ht1 / 100) \text{ x } 100 \text{ (\%)} \quad --- \text{(formula 2)}$$

According to the formulas 1 and 2 described above, the variation rate of the body weight (ΔBW%) and the variation rate of a volume of the circulating blood plasma (ΔCPV%) of the patient are measured by the measuring device 10 and are transmitted to the calculation device 11 to perform a predetermined calculation to obtain a parameter (PWI) (as will be described later).

During hemodialysis treatment, the calculation device 11 successively calculates a parameter relating the variation rate of the body weight to the variation rate of a volume of the circulating blood plasma (a variation rate of a blood benchmark), both obtained by the measuring device 10, in correlation with a dry-weight of a patient. As such a parameter to be calculated, an index PWI is used. PWI indicates how the variation (decrease) of a body weight of a patient affects the ultrafiltration on the blood concentration. Note that, although it is stated above that the calculation of PWI by the calculation means 11 is successively performed during hemodialysis treatment, the word "successively" indicates that a plurality of calculations are performed in the period from a start of the hemodialysis treatment to an end thereof, and also, calculations are performed at predetermined interval. Further, calculation of PWI by the calculation device 11 may be performed only once during the hemodialysis treatment. Only one calculation of PWI performed at the end of the hemodialysis treatment, for example, may confirm in real-time whether or not the body weight reaches the dry-weight by the hemodialysis treatment.

On the other hand, PWI is calculated by the formula (PWI = ΔCPV% /ΔBW%) where the variation rate of a volume of the circulating blood plasma (ΔCPV%) is divided by the variation rate of the body weight (ΔBW%), which proves that it is within an optimal range when the body weight of the patient reaches the dry-weight. Thus, the greater the value of PWI, the greater the value of the blood concentration rate in relation to decrease in the body weight due to the ultrafiltration, which can recognize that an interstitial fluid is not supplemented to the outside of blood vessels although water is removed from the blood by the ultrafiltration, and also recognize that, when the value of PWI is smaller, there is sufficient interstitial fluid in the outside of blood vessels even if water is removed from the blood by the ultrafiltration. Note that, the optimal value of PWI may vary as time passes during the hemodialysis treatment, and that the optimal value of PWI at the end of the hemodialysis treatment may vary depending on conditions of the hemodialysis.

The display means 12 is formed by a display device (not shown) provided in the dialysis device body 6, and displays a parameter (PWI) calculated by the calculation means 11 and also graphs (such as a line graph, for example) showing changes of PWI with time, which makes a medical staff such as a doctor recognize in real-time whether or not the ultrafiltration is optimally performed, and efficiently optimize the ultrafiltration during the hemodialysis treatment.

In addition, since the change of the calculated parameter (PWI) with time is graphically displayed, a medical staff can visually understand the change and trend of the parameter (PWI), and further the ultrafiltration can be optimized during the hemodialysis treatment. Note that, although the display means 12 graphically displays the parameter (PWI) in the present carrying-out mode, the display means 12 may display in real-time the numerical value of PWI successively calculated.

Moreover, prior to the hemodialysis treatment, when a target value of the ultrafiltration volume (UFV) is set, the body weight previous to the hemodialysis treatment is inputted into the input means 9, and the optimal range (2 to 5) of the parameter (PWI) at a hemodialysis treatment completion time is set, Ht2 at a hemodialysis treatment completion time can be predicted by a reverse calculation at the time when Ht1 is measured after starting the measurement of a blood benchmark. Accordingly, based on changes in the blood benchmark, it can be determined in advance whether or not it is possible to comfortably perform the hemodialysis treatment upon the patient, thus performing more optimized hemodialysis treatment.

The informing means 13 is used for informing a medical staff and the like in the vicinity of the informing means 13 of a state of a parameter (PWI) when the parameter (PWI) calculated by the calculation means 10 has deviated from an optimal range, and is formed by a speaker capable of outputting audio signals, for example, or a luminous source (such as an LED) capable of irradiating with light. The optimal range is to be set by inputting in advance from an input section of the dialysis device body 6, and is a range of parameters that are determined as ideal parameters (PWI) in relation to a target dry-weigh at each measuring time, in the hemodialysis treatment.

The control means 15 is used for controlling a dialyzing means (the ultrafiltration pump 8 according to the present carrying-out mode) so that the parameter (PWI) calculated by the calculation means 10 becomes a value within the optimal range when the parameter has deviated from the optimal range as stated above. Specifically, when a calculated parameter (PWI) has deviated from the optimal range, the control means 15 controls the ultrafiltration pump 8 to adjust an ultrafiltration rate so that the parameter (PWI) reaches a value within the optimal range to complete the hemodialysis treatment.

The storage means 14 is used for storing a parameter (PWI) calculated by the calculation means 11, and is formed by a memory provided in the dialysis device body 6, for example. The parameter (PWI) and its change with time stored in the storage means 14 can be displayed during another hemodialysis treatment (at a hemodialysis treatment for the identical patient following the prior treatment, or at a hemodialysis treatment for another patient), or displayed before or after the hemodialysis treatment. For example, displaying of the parameter and its change with time of the identical patient during a hemodialysis treatment following the prior treatment allows medical staffs to study a trend.of a mid-term or long term treatment and current conditions of the treatment Also, displaying of the parameter and its change with time during a hemodialysis treatment for another patient allows medical staffs to study a comparison between patients. Further, displaying of the parameter and its changes with time of the parameter before or after a hemodialysis treatment (for example, when the patient is waiting for the treatment in the vicinity of a hemodialysis apparatus (for example, on a bed which the patient lies on at the hemodialysis treatment) before a needle is inserted, or when the patient leaves the vicinity of the hemodialysis apparatus (for example, the bed which the patient lies on at the hemodialysis treatment) after the needle is extracted) allows medical staffs such as a doctor to explain to the patient the result of treatments with a comparison between current indications of the parameter and its changes with time and prior indications of them.

Furthermore, during the hemodialysis treatment for the identical patient, displaying of both a parameter (PWI) stored by the storage means 14 and a current parameter (PWI) calculated by the calculation means 11 on the display means 12 allows medical staffs to perceive the current conditions in relation to the optimal range of the parameter (PWI), thus performing more effective hemodialysis treatment.

Further, the hemodialysis apparatus may be provided with a guidance function for guiding an ideal treatment plan based on the current conditions perceived. In this case, when the parameter (PWI) indicates a value lower than that of the optimal range at the end of the current hemodialysis treatment, it is preferable to give a guidance to increase a volume of the ultrafiltration at the following hemodialysis treatment. Further, data stored in the storage means 14 maybe transmitted to an external terminal of the dialysis device body 6 through such as a network so that the data can be stored, displayed and compared with related data. In this case, data of patients can be easily shared, and the shared data of patients can be arranged in a data base, which allows a collective management.

According to the above-described carrying-out mode, since a parameter (PWI in concrete terms) relating a variation rate of a body weight to a variation rate of a blood benchmark and correlating with a dry-weight of a patient is calculated in real-time during a hemodialysis treatment, there is no need of manual calculation of the parameter. In addition, calculated parameter (PWI) is displayed in real-time, so that it is possible immediately to perceive in real-time the conditions of a patient during the hemodialysis treatment, to determine whether the conditions of the treatment is proper or improper, to predict changes and future conditions of the patient, and to confirm treatment results (effects).

Further, since all benchmarks necessary to calculate a parameter (PWI) can be obtained by using the input means 9 and sensors (such as a hematocrit sensor) provided in the existing hemodialysis apparatus, and display devices provided in the existing hemodialysis apparatus can be utilized as a display device and the like for displaying the parameter (PWI), the existing hemodialysis apparatus can be easily utilized, thus suppressing the manufacturing cost

As stated above, while the carrying-out mode of the present invention has been explained, the present invention is not restricted to the above-described carrying-out mode. For example, other parameters different from PWI may be used as far as the parameters correlate with a dry-weight of a patient, relating a variation rate of a body weight to a variation rate of a blood benchmark (not limited to a variation rate of a volume of circulating blood plasma), and are calculated during a hemodialysis treatment.

Further, when a parameter is calculated by using the variation rate of a volume of circulating blood plasma as the variation rate of the blood benchmark, another blood benchmark (such as a hemoglobin concentration, a blood serum total protein concentration) may be used instead of a hematocrit value. In this regard, hemoglobin identifies a pigment in red blood cells, so that the hemoglobin concentration is in correlation with the hematocrit value. Further, in some dialysis, some amount of protein leaks out to the dialysate side in the dialyzer, however, the amount of leaked protein is considered within a range of measurement error. Therefore, the blood serum total protein concentration may be used to measure the variation rate of a volume of circulating blood plasma as a blood benchmark. Note that, the hemoglobin concentration and the blood serum total protein concentration may be measured by utilizing an optical device, an ultrasonic device or the like.

Further, with regard to disturbance elements having an influence on the variation rate of a body weight and the variation rate of a blood benchmark (such as changes in concentration of dialysate, changes in blood temperature, changes in blood flow rate, elements such as dosing a supplemental fluid, a high sodium fluid and the like exerting influence upon blood, and elements such as intake of food, excretion, fluid supplement exerting influence upon a body weight), the hemodialysis apparatus may have a structure including input or store of disturbance elements, informing of detection of disturbance elements, or suspension or correction at the calculation of parameter.

INDUSTRIAL POTENTIAL OF UTILIZATION

The present invention may be applied to other mode of hemodialysis apparatus with other functions or no accompanying function, as far as the hemodialysis apparatus calculates during a hemodialysis treatment a parameter relating the variation rate of a body weight to the variation rate of a blood benchmark and correlating with the dry-weight of a patient as described above.

BRIEF DESCRIPTION OF THE DRAWINGS

[Fig. 1] A schematic diagram illustrating the entire hemodialysis apparatus according to the carrying-out mode of the present invention.

[Fig. 2] A schematic diagram illustrating the internal mechanical structure of the hemodialysis device according to the carrying-out mode of the present invention.

[Fig. 3] A schematic diagram illustrating the internal electrical structure and the like of the hemodialysis device according to the carrying-out mode of the present invention.

DESCRIPTION OF THE SYMBOLS

1    Blood circuit
1a   Arterial blood circuit
1b   Venous blood circuit
2    Dialyzer (dialysis treatment means)
3    Blood pump
4    Venous drip chamber
5    Hematocrit sensor
6    Dialysis device body
7    Dialysate supplying device
8    Ultrafiltration pump
9    Input means
10   Measuring means
11   Calculation means
12   Display means
13   Informing means
14   Storage means
15   Control means
16   Ultrafiltration volume calculation means

## Claims

1. A hemodialysis apparatus comprising:

    a dialysis treatment means for performing hemodialysis and ultrafiltration upon an extracorporeally-circulating blood of a patient to conduct a hemodialysis treatment; and
    a measuring means (10) for measuring a variation rate ($\Delta BW\%$) of a body weight of said patient and a variation rate ($\Delta CPV\%$) of a predetermined blood benchmark during said hemodialysis treatment,

    **characterized in that**
    a calculation means (11) is provided for calculating a parameter (PWI) by relating said variation rate ($\Delta BW\%$) of said body weight to said variation rate ($\Delta CPV\%$) of said blood benchmark obtained by said measuring means (10) during said hemodialysis treatment,
    wherein said parameter (PWI) correlates with a dry-weight of said patient;
    wherein said parameter (PWI) is calculated by relating
    said variation rate of said body weight ($\Delta BW\%$), which is obtained by relating said body weight (BW1) of said patient prior to said hemodialysis treatment and a volume of ultrafiltration (UFV), and
    said variation rate ($\Delta CPV\%$) of said blood benchmark, which is obtained from a concentration value (Ht1, Ht2) of said extracorporeally-circulating blood.

2. The hemodialysis apparatus as set forth in claim 1, **characterized in that** a variation rate ($\Delta CPV\%$) of a volume of circulating blood plasma of said extracorporeally-circulating blood is calculated from said concentration value (Ht1, Ht2) of blood, and
    said variation rate ($\Delta CPV\%$) of said volume of circulating blood plasma is a variation rate of said blood benchmark.

3. The hemodialysis apparatus as set forth in any one of claim 1 to claim 3, **characterized by** a display means (12) for displaying said parameter calculated by said calculation means (11).

4. The hemodialysis apparatus as set forth in claim 3, **characterized in that** said display means (12) graphically displays a change of said parameter (PWI) with respect to time as calculated by said calculation means (11).

5. The hemodialysis apparatus as set forth in any one of claim 1 to claim 4, **characterized by** an informing means (13) for setting an optimal range of said parameter (PWI) and informing when a value of said parameter (PWI) as calculated by said calculation means (11) deviates from said optimal range of said parameter (PWI).

6. The hemodialysis apparatus as set forth in any one of claim 1 to claim 5, **characterized by** a control means (15) for setting an optimal range of said parameter (PWI) and controlling said dialysis treatment means so that a value of said parameter (PWI) becomes a value within said optimal range, when a value of said parameter (PWI) as calculated by said calculation means (11) deviates from said optimal range of said parameter (PWI).

7. The hemodialysis apparatus as set forth in any one of claim 1 to claim 6, **characterized by** a storage means (14) for storing said parameter (PWI) as calculated by said calculation means (11), so that said parameter (PWI) stored by said storage means (14) or a change of said stored parameter (PWI) with respect to time can be displayed during other hemodialysis treatment or before or after said hemodialysis treatment.

8. The hemodialysis apparatus as set forth in claim 7, **characterized in that** said storage means (14) connected to an external terminal is capable of transmitting said parameter (PWI) as calculated by said calculation means (11) to said external terminal.

## Patentansprüche

1. Hämodialyse-Vorrichtung, aufweisend:

    eine Dialyse-Behandlungseinrichtung zum Durchführen einer Hämodialyse und einer Ultrafiltration bei einem extrakorporeal zirkulierenden Blut eines Patienten, um eine Hämodialyse-Behandlung durchzuführen; und
    eine Messeinrichtung (10) zum Messen einer Variationsrate ($\Delta BW\%$) eines Körpergewichtes des Patienten und einer Variationsrate ($\Delta CPV\%$) eines vorbestimmten Blutbezugswertes während der Hämodialyse-Behandlung,

**dadurch gekennzeichnet, dass**
eine Berechnungseinrichtung (11) vorgesehen ist, um einen Parameter (PWI) zu berechnen, und zwar **dadurch**, dass die Variationsrate (ΔBW%) des Körpergewichts zur Variationsrate (ΔCPV%) des Blutbezugswertes in Bezug gesetzt wird, der durch die Messeinrichtung (10) während der Hämodialyse-Behandlung erhalten wird, wobei der Parameter (PWI) mit einem Trockengewicht des Patienten korreliert; wobei der Parameter (PWI) **dadurch** berechnet wird, dass in Bezug gesetzt werden:

die Variationsrate des Körpergewichtes (ΔBW%), die **dadurch** erhalten wird, dass das Körpergewicht (BW1) des Patienten vor der Hämodialyse-Behandlung und ein Ultrafiltrationsvolumen (UFV) in Bezug gesetzt werden, und

die Variationsrate (ΔCPV%) des Blutbezugswertes, die aus einem Konzentrationswert (Ht1, Ht2) des extrakorporeal zirkulierenden Blutes erhalten wird.

2. Hämodialyse-Vorrichtung nach Anspruch 1, **dadurch gekennzeichnet, dass** eine Variationsrate (ΔCPV%) eines Volumens von zirkulierendem Blutplasma des extrakorporeal zirkulierenden Blutes aus dem Konzentrationswert (Ht1, Ht2) des Blutes berechnet wird, und die Variationsrate (ΔCPV%) des Volumens von zirkulierendem Blutplasma eine Variationsrate des Blutbezugswertes ist.

3. Hämodialyse-Vorrichtung nach einem der Ansprüche 1 bis 3, **gekennzeichnet durch** eine Anzeigeeinrichtung (12) zum Anzeigen der **durch** die Berechnungseinrichtung (11) berechneten Parameter.

4. Hämodialyse-Vorrichtung nach Anspruch 3, **dadurch gekennzeichnet, dass** die Anzeigeeinrichtung (12) eine Änderung des Parameters (PWI) bezüglich der Zeit graphisch anzeigt, wie durch die Berechnungseinrichtung (11) berechnet.

5. Hämodialyse-Vorrichtung nach einem der Ansprüche 1 bis 4, **gekennzeichnet durch** eine Informationseinrichtung (13), um einen optimalen Bereich des Parameters (PWI) festzulegen und um zu informieren, wenn ein Wert des Parameters (PWI), wie **durch** die Berechnungseinrichtung (11) berechnet, von dem optimalen Bereich des Parameters (PWI) abweicht.

6. Hämodialyse-Vorrichtung nach einem der Ansprüche 1 bis 5, **gekennzeichnet durch** eine Steuereinrichtung (15), um einen optimalen Bereich des Parameters (PWI) festzulegen und um die Dialyse-Behandlungseinrichtung so zu steuern, dass ein Wert des Parameters (PWI) zu einem Wert innerhalb des optimalen Bereiches wird, wenn ein Wert des Parameters (PWI), wie berechnet **durch** die Berechnungseinrichtung (11), von dem optimalen Bereich des Parameters (PWI) abweicht.

7. Hämodialyse-Vorrichtung nach einem der Ansprüche 1 bis 6, **gekennzeichnet durch** eine Speichereinrichtung (14) zum Speichern des Parameters (PWI), wie durch die Berechnungseinrichtung (11) berechnet, so dass der Parameter (PWI), der **durch** die Speichereinrichtung (14) gespeichert ist, oder eine Änderung des gespeicherten Parameters (PWI) bezogen auf die Zeit während einer anderen Hämodialyse-Behandlung oder vor oder nach der Hämodialyse-Behandlung angezeigt werden kann.

8. Hämodialyse-Vorrichtung nach Anspruch 7, **dadurch gekennzeichnet, dass** die Speichereinrichtung (14), die an einen externen Anschluss angeschlossen ist, fähig ist, den Parameter (PWI), wie berechnet durch die Berechnungseinrichtung (11), an den externen Anschluss zu übertragen.

**Revendications**

1. Appareil d'hémodialyse comprenant :

des moyens de traitement de dialyse pour réaliser l'hémodialyse et l'ultra-filtration suite à une circulation extracorporelle du sang d'un patient pour réaliser un traitement d'hémodialyse ; et
des moyens de mesure (10) pour mesurer un taux de variation (ΔBW%) d'un poids corporel dudit patient et un taux de variation (ΔCPV%) d'une référence de sang prédéterminée pendant ledit traitement d'hémodialyse,

**caractérisé en ce que** :

on prévoit des moyens de calcul (11) pour calculer un paramètre (PWI) en reliant ledit taux de variation ($\Delta$BW%) dudit poids corporel audit taux de variation ($\Delta$CPV%) de ladite référence de sang obtenue par lesdits moyens de mesure (10) pendant ledit traitement d'hémodialyse,

dans lequel ledit paramètre (PWI) est en corrélation avec un poids sec dudit patient ;

dans lequel ledit paramètre (PWI) est calculé en reliant :

ledit taux de variation dudit poids corporel ($\Delta$BW%) qui est obtenu en reliant ledit poids corporel (BW1) dudit patient avant ledit traitement d'hémodialyse et un volume d'ultra-filtration (UFV), et

ledit taux de variation ($\Delta$CPV%) de ladite référence de sang qui est obtenue à partir d'une valeur de concentration (Ht1, Ht2) dudit sang de circulation extracorporelle.

2. Appareil d'hémodialyse selon la revendication 1, **caractérisé en ce qu'**un taux de variation ($\Delta$CPV%) d'un volume de plasma sanguin périphérique dudit sang de circulation extracorporelle est calculé à partir de ladite valeur de concentration (Ht1, Ht2) du sang, et
   ledit taux de variation ($\Delta$CPV%) dudit volume de plasma sanguin périphérique est un taux de variation de ladite référence de sang.

3. Appareil d'hémodialyse selon l'une quelconque des revendications 1 à 3, **caractérisé par** des moyens d'affichage (12) pour afficher ledit paramètre calculé par lesdits moyens de calcul (11).

4. Appareil d'hémodialyse selon la revendication 3, **caractérisé en ce que** lesdits moyens d'affichage (12) affiche graphiquement un changement dudit paramètre (PWI) par rapport au temps tel que calculé par lesdits moyens de calcul (11).

5. Appareil d'hémodialyse selon l'une quelconque des revendications 1 à 4, **caractérisé par** des moyens d'information (13) pour régler une plage optimale dudit paramètre (PWI) et informer lorsqu'une valeur dudit paramètre (PWI) telle que calculée par lesdits moyens de calcul (11) dévie de ladite plage optimale dudit paramètre (PWI).

6. Appareil d'hémodialyse selon l'une quelconque des revendications 1 à 5, **caractérisé par** des moyens de commande (15) pour régler une plage optimale dudit paramètre (PWI) et commander lesdits moyens de traitement de dialyse de sorte qu'une valeur dudit paramètre (PWI) devient une valeur dans ladite plage optimale, lorsqu'une valeur dudit paramètre (PWI) tel que calculé par lesdits moyens de calcul (11) dévie de ladite plage optimale dudit paramètre (PWI).

7. Appareil d'hémodialyse selon l'une quelconque des revendications 1 à 6, **caractérisé par** des moyens de stockage (14) pour stocker ledit paramètre (PWI) tel que calculé par lesdits moyens de calcul (11), de sorte que ledit paramètre (PWI) stocké par lesdits moyens de stockage (14) ou un changement dudit paramètre stocké (PWI) par rapport au temps peut être affiché pendant un autre traitement d'hémodialyse ou avant ou après ledit traitement d'hémodialyse.

8. Appareil d'hémodialyse selon la revendication 7, **caractérisé en ce que** lesdits moyens de stockage (14) raccordés à un terminal externe peuvent transmettre ledit paramètre (PWI) tel que calculé par lesdits moyens de calcul (11) audit terminal externe.

【Fig.1】

[Fig. 2]

[Fig. 3]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5938938 A